# FASCICULE DE BREVET EUROPEEN

(11) **EP 2 928 499 B1**
(45) Date de publication et mention de la délivrance du brevet: **31.01.2018**
(21) Numéro de dépôt: 13815054.5
(22) Date de dépôt: 05.12.2013
(51) Int. Cl.: A61K 47/10, A61K 47/32, A61K 31/57, A61K 9/10, A61K 9/20

(54) **DISPERSION SOLIDE D'UN MODULATEUR SELECTIF DU RECEPTEUR A LA PROGESTERONE**
FESTE DISPERSION EINES SELEKTIVEN MODULATORS DES PROGESTERONREZEPTORS
SOLID DISPERSION OF A SELECTIVE MODULATOR OF THE PROGESTERONE RECEPTOR

(30) Priorité: 06.12.2012 FR 1261721
(43) Date de publication de la demande: 14.10.2015
(73) Titulaire: Laboratoire HRA Pharma, 75003 Paris (FR)
(72) Inventeur: BATTUNG, Florian, 75010 Paris (FR); JUVIN, Pierre-Yves, 44100 Nantes (FR); HECQ, Jérôme, 33360 Camblanes Et Meynac (FR); COLIN, Aude, 33100 Bordeaux (FR)
(74) Mandataire: Cabinet Becker et Associés
(86) Numéro de dépôt international: PCT/FR2013/052952
(87) Numéro de publication internationale: WO 2014/087106

(56) Documents cités:
- WO-A2-2008/083192
- US-A1- 2010 144 692
- DATABASE WPI Week 201171 Thomson Scientific, London, GB; AN 2011-J86758 XP002706347, -& CN 102 107 007 A (GUANGZHOU REGENEX CORP) 29 juin 2011 (2011-06-29)
- DATABASE WPI Week 201311 Thomson Scientific, London, GB; AN 2012-N57639 XP002707247, -& CN 102 600 001 A (NAT RES INST FAMILY PLANNING) 25 juillet 2012 (2012-07-25)

## Description

### Domaine de l'invention

La présente invention concerne une nouvelle forme galénique d'un modulateur sélectif du récepteur à la progestérone (SPRM), plus précisément, une dispersion solide, et des compositions pharmaceutiques contenant ladite forme galénique.

### Arrière-plan technologique de l'invention

L'ulipristal acétate (en abrégé UPA) correspond à la 17α-acétoxy-11β-[4-(N,N-diméthylamino)-phényl]-19-norprégna- 4, 9-diène-3,20-dione (nomenclature IUPAC) et possède la formule chimique suivante :

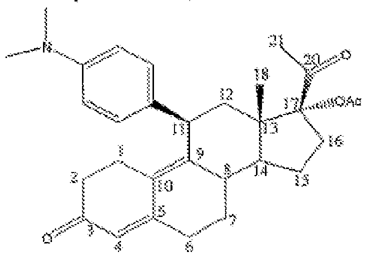

Sa synthèse est décrite, entre autres, dans le brevet EP 0 422 100 et dans la demande de brevet EP 1 602 662.

L'ulipristal acétate est un modulateur synthétique sélectif des récepteurs de la progestérone (SPRM). Par son action sur le récepteur de la progestérone, l'ulipristal acétate est capable d'exercer une action contraceptive en inhibant ou en retardant l'ovulation. Des études cliniques ont montré que l'ulipristal acétate, administré en une dose unique de 30 mg, permet d'éviter une grossesse non désirée quand il est administré dans les 120 heures suivant un rapport sexuel non ou mal protégé (Glasier et al, Lancet. 2010, 375(9714):555-62 ; Fine et al, Obstet Gynecol. 2010, 115:257-63). L'ulipristal acétate a été ainsi autorisé en tant que contraceptif d'urgence et est commercialisé sous le nom commercial EllaOne® en Europe.

D'autres applications thérapeutiques de l'ulipristal acétate ont été proposées dans l'état de la technique. Des essais cliniques récents ont montré que l'administration chronique d'ulipristal acétate (à raison de 5mg ou 10 mg par jour) permet de réduire de manière significative les symptômes associés aux fibromes utérins et fournit un bénéfice thérapeutique supérieur à celui du traitement de référence, à savoir l'acétate de leuprolide (Donnez et al., N Engl J Med. 2012; 366(5):421-32.). Sur la base de ces essais cliniques, l'Agence Européenne du Médicament (EMA) a autorisé, en février 2012, la spécialité Esmya ® (5mg d'ulipristal acétate) pour le traitement pré-opératoire des symptômes associés aux fibromes utérins.

Les compositions pharmaceutiques actuellement commercialisées comportent de l'ulipristal acétate sous une forme micronisée.

La spécialité Esmya® se présente sous la forme d'un comprimé non-pelliculé comprenant 5 mg d'ulipristal acétate micronisé associés aux excipients suivants : cellulose microcristalline, mannitol, croscarmellose sodique, talc et stéarate de magnésium.

EllaOne® se présente, quant à elle, sous la forme d'un comprimé non-pelliculé comprenant 30 mg d'ulipristal acétate micronisé et les excipients suivants : lactose monohydraté, povidone K30, croscarmellose sodique et stéarate de magnésium.

Des compositions pharmaceutiques analogues ont été également décrites dans les demandes internationales WO 2010/066749 et WO 2008/083192. La mise au point de nouvelles formes galéniques adaptées pour l'administration des modulateurs sélectifs du récepteur à la progestérone tels que l'ulipristal acétate demeure un enjeu majeur pour leurs utilisations thérapeutique et contraceptive.

A cet égard, il existe, à l'heure actuelle, un besoin de formulations pharmaceutiques alternatives comprenant un modulateur sélectif du récepteur à la progestérone, tels que l'ulipristal acétate, et présentant des propriétés de libération et une biodisponibilité adaptées.

### Résumé de l'invention

L'invention a pour objet une dispersion solide telle que définie dans les revendications. Le principe actif est choisi parmi le groupe constitué par la 17α-acétoxy-11β-[4-N-méthylamino-phényl]-19-norprégna-4,9-diène-3,20-dione, la 17α-acétoxy-11β-[4-amino-phényl]-19-norprégna-4,9-diène-3,20-dione, l'ulipristal acétate et leurs mélanges. L'excipient polymérique acceptable sur le plan pharmaceutique est choisi parmi le groupe constitué par polyvinylpyrrolidones, les copolymères de N-vinyl-2-pyrrolidone, et leurs mélanges.

La dispersion solide selon l'invention peut être caractérisée par une ou plusieurs des caractéristiques suivantes :
- le principe actif est l'ulipristal acétate,
- l'excipient polymérique est choisi parmi le groupe constitué par les polyvinylpyrrolidones, les copolymères de N-vinyl-2-pyrrolidone et d'acétate de vinyle, et leurs mélanges,
- Le rapport massique « excipient polymérique/principe actif » est compris dans une gamme allant de 1 à 50, de préférence de 1 à 30,
- la dispersion solide comprend en outre un surfactant, le rapport massique « principe actif/surfactant » pouvant être compris dans une gamme allant de 0,5 à 10 et/ou ledit surfactant pouvant être un sel de dodécylsulfate, de préférence le dodécylsulfate de sodium, et
- la dispersion solide présente sous la forme d'un dépôt à la surface d'un véhicule acceptable sur le plan pharmaceutique.

La présente invention a également pour objet une méthode de préparation d'une dispersion solide telle que décrite précédemment caractérisée en ce qu'elle comprend les étapes consistant à :
a) préparer une solution comprenant l'excipient polymérique et le principe actif dans un solvant, puis éliminer ledit solvant de manière à obtenir la dispersion solide ; ou
b) préparer un mélange comprenant l'excipient polymérique à l'état fondu et le principe actif puis solidifier ledit mélange de manière à obtenir la dispersion solide.

Un objet supplémentaire selon l'invention est une composition pharmaceutique comprenant une dispersion solide telle que définie précédemment et un excipient acceptable sur le plan pharmaceutique, de préférence choisi parmi le groupe constitué par un diluant, un liant, un agent d'écoulement, un lubrifiant, un agent désintégrant et leurs mélanges. De préférence, la composition pharmaceutique comprend de 1 mg à 100 mg, de préférence de 1 mg à 40 mg de principe actif par unité de dose et est est adaptée à une administration par voie orale. Elle peut se présenter sous la forme d'une poudre, d'une granule, d'un comprimé pelliculé ou non pelliculé, ou d'une gélule.

La présente invention a pour objet une dispersion solide ou une composition pharmaceutique telles que décrites précédemment pour une utilisation en tant que contraceptif, en particulier en tant que contraceptif d'urgence ou contraceptif régulier.

Enfin, l'invention a également pour objet une dispersion solide ou une composition pharmaceutique telles que décrites précédemment, pour une utilisation dans le traitement ou la prévention d'un trouble gynécologique, de préférence affectant l'utérus, par exemple les fibromes utérins.

### Figures

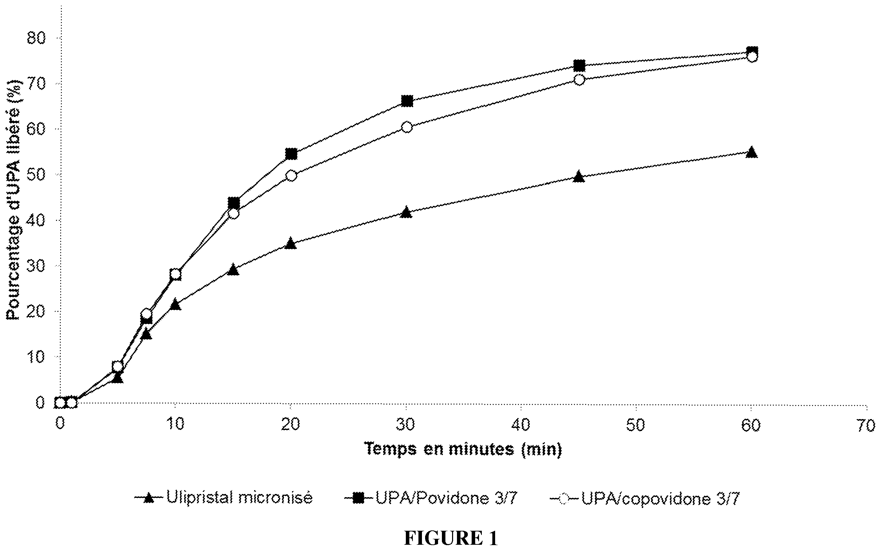

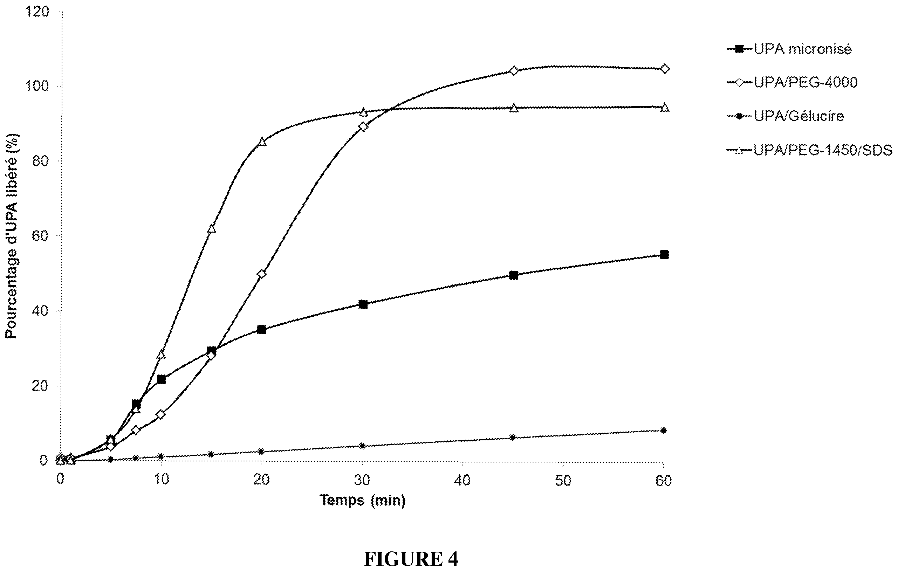

La **Figure 1** montre les courbes de dissolution *in vitro* pour différentes dispersions solides obtenues par la méthode dite par évaporation de solvant (voir Exemple 1 ci-après) : UPA/povidone 3/7 (carré plein), UPA/copovidone 3/7 (rond vide), Expérience témoin : UPA micronisé (triangle plein). Ordonnées : pourcentage d'UPA libéré (%), abscisse : temps en minutes.

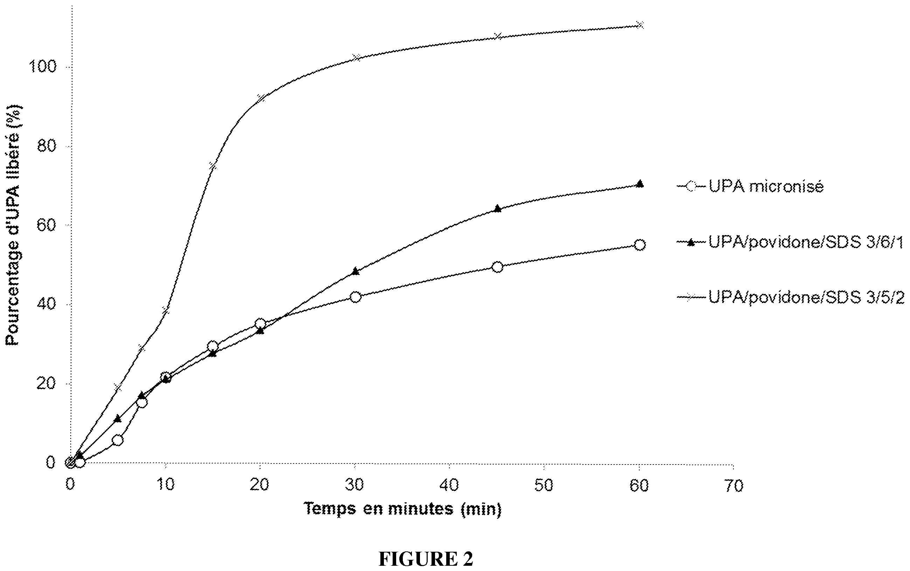

La **Figure 2** montre les courbes de dissolution *in vitro* pour différentes dispersions solides (voir Exemple 1 ci-après) : UPA/copovidone/SDS 3/6/1 (carré plein), UPA/povidone/SDS 3/6/1 (triangle plein), UPA/povidone/SDS 3/5/2 (croix), Expérience témoin : UPA micronisé (rond vide). Ordonnées : pourcentage d'UPA libéré (%), abscisse : temps en minutes.

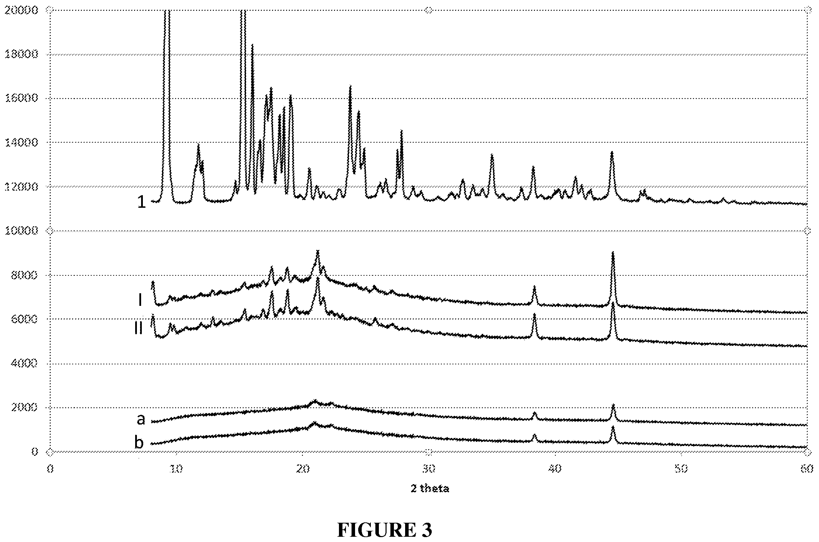

La **Figure 3** montre les spectres de diffraction sur poudre par rayons X de:
   - l'UPA non micronisé (courbe 1),
   - la dispersion solide UPA/povidone/SDS 3/5/2 avant (courbe I) et après stockage pendant 1 mois (courbe II) et
   - la dispersion solide UPA/povidone/SDS 3/5/1 avant (courbe a) et après stockage pendant 1 mois (courbe b)..
**La** **Figure 4** montre les courbes de dissolution *in vitro* pour différentes dispersions solides obtenues par la méthode par fusion (voir Exemple 1 ci-après) : UPA/PEG-4000 (losage vide), UPA/PEG-1450/SDS (triangle vide), UPA/gelucire® (rond plein), Expérience témoin : UPA micronisé (carré plein). Ordonnées : pourcentage d'UPA libéré (%), abscisse : temps en minutes.

### Description détaillée de l'invention

### Dispersion solide selon l'invention et procédé de préparation

Le Demandeur a montré qu'il est possible d'améliorer de manière significative le profil de dissolution *in vitro* de l'ulipristal acétate (ci-après UPA) grâce à une technologie de dispersion solide. A cet égard, l'exemple 1 de la présente demande montre qu'une dispersion solide comprenant de l'ulipristal acétate dispersé dans une matrice polymérique, par exemple de type povidone ou copovidone, présente des propriétés de dissolution *in vitro* de l'UPA - en particulier une vitesse de dissolution - bien supérieure à celle de l'UPA micronisé. Cette amélioration des propriétés de dissolution *in vitro* de l'UPA apportée par la technique de dispersion solide devrait se traduire *in vivo* par une nette augmentation de la biodisponibilité de l'UPA. Il est attendu que la dispersion solide permette de diminuer la dose d'UPA à administrer au patient pour obtenir l'effet thérapeutique ou contraceptif recherché, améliorant ainsi la sécurité, en particulier l'innocuité, des compositions pharmaceutiques à base d'UPA.

Ainsi, un premier objet de la présente invention est une nouvelle forme galénique, plus précisément, une dispersion solide comprenant :
- un excipient polymérique, et
- un principe actif choisi parmi le groupe constitué par les modulateurs sélectifs des récepteurs à la progestérone, leurs métabolites et leurs mélanges.
Par « dispersion solide», on entend un système à l'état solide à température ambiante comprenant des particules de principe actif dispersées, de préférence de manière homogène, dans une matrice solide. Dans le cadre de la présente invention, la matrice solide comprend un excipient de nature polymérique c'est-à-dire un polymère ou un mélange de polymères acceptables sur le plan pharmaceutique. La dispersion solide peut comprendre, en plus du principe actif et de l'excipient polymérique, un ou plusieurs excipients additionnels, qui sont, soit un constituant de la matrice, soit dispersés dans ladite matrice. La dispersion solide peut être obtenue par toute méthode connue de l'homme du métier. La présente description propose plus loin plusieurs méthodes adaptées à la préparation d'une dispersion solide selon l'invention.
On entend par « excipient polymérique » tout polymère ou mélange de polymères adaptés à une utilisation en tant que matrice de dispersion solide et acceptables sur le plan pharmaceutique.
L'excipient polymérique peut être choisi parmi :
▪ les polymères présentant une température de transition vitreuse (Tg) inférieure à 200°C, de préférence inférieure à 100°C, et leurs mélanges, et
▪ les polymères solubles dans l'eau ou dans un solvant organique, ledit solvant pouvant être le dichlorométhane, l'acétone, un alcool, par exemple le méthanol, l'éthanol, l'isopropanol, ou encore un ester tel que l'acétate d'éthyle
L'excipient polymérique est choisi parmi le groupe constitué par les polymères et copolymères de N-vinyl-2-pyrrolidone, et les mélanges desdits polymères et copolymères.

Comme cela est illustré dans les exemples, les polymères à base de N-vinyl-pyrrolidone présentent une capacité de charge élevée en principe actif.
Ainsi, dans un mode de réalisation encore plus préféré, l'excipient polymérique est choisi parmi le groupe constitué par les polymères et les copolymères de N-vinyl-2-pyrrolidone, et leurs mélanges. De préférence, ces polymères sont non-réticulés. En d'autres termes, de préférence, lesdits polymères n'englobent pas les crospovidones.
Les polymères de N-vinyl-2-pyrrolidone correspondent à des homopolymères de N-vinylpyrrolidone, également appelés dans la littérature polyvinylpyrrolidone, povidone ou PVP. Des polyvinylpyrrolidones adaptées pour la mise en oeuvre de la présente invention peuvent avoir un poids moléculaire allant de 10³ à 10⁷ g.mol⁻¹, de préférence de 10 000 à 300 000 g.mol⁻¹, et de manière encore plus préférée, de 10 000 à 100 000 g.mol⁻¹. Les polyvinylpyrrolidones adaptées pour la mise en oeuvre de l'invention sont par exemple les polyvinylpyrrolidones solubles dans l'eau ou dans un alcool.
Un exemple de PVP adapté est la PVP commercialisée sous le nom Plasdone® K29-32 par la société ISP qui a un poids moléculaire moyen d'environ 58 000 g.mol⁻¹ et une valeur K allant de 29 à 32.
Les copolymères de N-vinyl-2-pyrrolidone correspondent à des polymères comprenant au moins deux types de monomères distincts, dont des monomères de type N-vinyl-2-pyrrolidone. Les copolymères de N-vinyl-2-pyrrolidone englobent, par exemple, les copolymères de N-vinyl-2-pyrrolidone et d'acétate de vinyle. Les copolymères N-vinyl-2-pyrrolidone et d'acétate de vinyle sont également désignés dans l'état de la technique par le terme copovidone. Une copovidone adaptée à la mise en oeuvre de la présente invention peut comprendre un ratio molaire N-vinyl-2-pyrrolidone/acétate de vinyle allant de 3/7 à 7/3, de préférence de 5/5 à 7/3, par exemple un ratio molaire d'environ 6/4. Le principe actif présent dans la dispersion solide est sélectionné parmi le groupe constitué par la 17α-acétoxy-11β-[4-N-méthylamino-phényl]-19-norprégna-4,9-diène-3,20-dione, la 17α-acétoxy-11β-[4-amino-phényl]-19-norprégna-4,9-diène-3,20-dione, l'ulipristal acétate et leurs mélanges. La dispersion solide comprend :
- un principe actif choisi parmi le groupe constitué par la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, l'ulipristal acétate et leurs mélanges, de préférence l'ulipristal acétate, et
- un excipient polymérique choisi parmi le groupe constitué par les polyéthylène glycols, les polymères et les copolymères de N-vinylpyrrolidone, et les combinaison de ceux-ci..
De préférence, l'excipient polymérique est choisi parmi le groupe constitué par les polyvinylpyrrolidones, les copolymères de N-vinylpyrrolidone, tels que les copovidones, et leurs combinaisons.
Le rapport massique entre l'excipient polymérique et le principe actif dans la dispersion solide selon l'invention est généralement compris dans une gamme allant de 1 à 100, de préférence de 1 à 80, de manière plus préférée de 1 à 60, et de manière encore plus préférée de 1 à 50. Un rapport massique dans une gamme allant de 1 à 50 englobe un rapport massique dans une gamme allant de 1 à 10, de 10 à 20, de 20 à 25, de 25 à 30, de 30 à 40, de 40 à 50. En particulier, le rapport massique peut être compris dans une gamme allant de 1 à 30.
Dans certains modes de réalisation, le rapport massique « excipient polymérique/principe actif » est compris dans une gamme allant de 1 à 20, de préférence de 1,5 à 10, voire de 1,5 à 5. De tels rapports massiques peuvent être obtenus, par exemple, pour une dispersion solide préparée par la méthode dite par évaporation de solvant.
Dans d'autres modes de réalisation, le rapport massique «excipient polymérique/principe actif » est compris dans une gamme allant de 20 à 50, de préférence de 20 à 30. Un tel rapport massique peut être obtenu, par exemple, pour des dispersions solides préparées par la méthode dite « par fusion ».

Les méthodes de préparation des dispersions solides par fusion et par évaporation de solvant sont décrites plus loin dans la présente description.
Le rapport massique « excipient polymérique/principe actif » peut être choisi en fonction de son utilisation finale. A titre d'exemple, lorsque la dispersion solide est intégrée dans un comprimé oral, le rapport massique « excipient polymérique/principe actif » est choisi de manière à ce que la taille du comprimé final permette son administration par voie orale au patient.

Dans certains modes de réalisation, la dispersion solide selon l'invention peut comprendre un excipient additionnel. Cet excipient additionnel peut permettre de potentialiser l'action de l'excipient polymérique sur les propriétés de dissolution ou d'améliorer la biodisponibilité de l'ulipristal acétate. Cet excipient additionnel peut être un constituant de la matrice de la dispersion solide ou y être dispersé. Dans certains modes de réalisation, cet excipient est choisi parmi les surfactants acceptables sur un plan pharmaceutique. De tels surfactants englobent, sans y être limités, la simethicone, la triéthanolamine, les dérivés polysorbate tels que le tween® 20 ou le tween® 40, les poloxamers, les alcools gras tels que l'alcool laurylique ou l'alcool cétylique ou encore les alkylsulfates.
Le surfactant peut être, en particulier, choisi parmi les sels d'alkylsulfates en C₈-C₂₀, de préférence en C₁₀-C₁₄ et leurs mélanges. De préférence, le surfactant est choisi parmi les sels de dodécylsulfate, de préférence, les sels alcalins ou alcalino-terreux de celui-ci tel qu'un sel de sodium, de magnésium ou de calcium. Un surfactant particulièrement préféré est le SDS c'est-à-dire le dodécyl-sulfate de sodium.
A titre d'exemple, la dispersion solide selon l'invention peut comprendre :
▪ un principe actif choisi parmi le groupe constitué par l'ulipristal acétate, la 17α-acétoxy-11β-[4-N-méthylamino-phényl)-19-norprégna-4,9-diène-3,20-dione, la 17α-acétoxy-11β-[4-amino-phényl)-19-norprégna-4,9-diène-3,20-dione, et leurs mélanges, de préférence l'ulipristal acétate,
▪ un excipient polymérique choisi parmi les povidones, les copovidones et leurs mélanges, et
▪ un surfactant solide, de préférence le SDS.

Le rapport massique entre le principe actif et le surfactant est généralement compris dans une gamme allant de 0,5 à 10, de préférence, de 0,5 à 4. Un rapport massique «principe actif/surfactant» adapté est, par exemple, un rapport massique d'environ 3.

Dans d'autres modes de réalisation, la dispersion solide est dépourvue de tout surfactant et plus généralement de tout excipient supplémentaire à l'excipient polymérique. Ainsi, dans certains modes de réalisation, la dispersion solide peut être constituée par le principe actif et l'excipient polymérique, uniquement.

Dans certains modes de réalisation, le principe actif est présent majoritairement sous une forme amorphe dans la dispersion solide selon l'invention.
On entend par « principe actif présent majoritairement sous une forme amorphe » le fait qu'au moins 80% du principe actif présent dans la dispersion solide est sous forme amorphe. En d'autres termes, la dispersion solide contient au plus 20% de principe actif sous forme cristalline, les pourcentages se référant à la quantité totale de principe actif présente dans la dispersion solide.
Le principe actif peut être présent essentiellement sous une forme amorphe dans la dispersion solide selon l'invention. En d'autres termes, au moins 95% du principe actif présent dans la dispersion solide peut être sous forme amorphe. « Au moins 95% de principe actif sous forme amorphe » comprend au moins 96%, au moins 97%, au moins 98%, au moins 98,5%, au moins 99%, au moins 99,2%, au moins 99,4%, au moins 99,6%, au moins 99,8%, au moins 99,9% de principe actif sous forme amorphe.
Afin que le principe actif soit présent majoritairement sous une forme amorphe, il est préférable que la dispersion solide comprenne moins de 20% en poids de surfactant, de préférence, moins de 15% en poids de surfactant, voire au plus 10% en poids de surfactant.

En fonction de son mode préparation, la dispersion solide selon l'invention peut se présenter sous la forme d'une poudre, éventuellement micronisée, ou sous la forme d'un dépôt sur un véhicule acceptable sur le plan pharmaceutique. Le véhicule acceptable sur le plan pharmaceutique peut être un diluant tel que la cellulose microcristalline ou les monosaccharides tels que le lactose ou le mannitol. Des exemples supplémentaires de diluants sont fournis plus loin dans la présente description.

On entend par « une poudre micronisée » une poudre dont la taille des particules est caractérisée par une d90 inférieure à 50 µm, ce qui signifie qu'au moins 90% des particules de la poudre ont une taille inférieure à 50 µm, la taille des particules se référant à leur plus grande dimension.

Comme cela est montré dans les exemples, la dispersion solide selon l'invention présente des propriétés améliorées de dissolution *in vitro* du principe actif. Dans certains modes de réalisation, la dispersion solide selon l'invention est caractérisée en ce qu'au moins 60% du principe actif qu'elle contient est libéré en 45 minutes lorsque ladite dispersion solide est soumise à un test de dissolution *in vitro.*
Le test de dissolution *in vitro* peut être réalisé à l'aide de tout dispositif de dissolution à pales disponible dans le commerce. L'exemple 1 ci-après présente des conditions de mise en oeuvre pour la détermination de la vitesse de dissolution *in vitro* d'une dispersion solide selon l'invention. Brièvement, une quantité de dispersion solide représentant 30 mg de principe actif est disposée dans une gélule en gélatine. Cette gélule est ensuite placée dans 900 ml d'un milieu tamponné à pH gastrique, comprenant 0,1% de SDS, à 37±0,5 °C et soumis à une agitation de 50 tours par minute (rpm) (vitesse de rotation des pales du dispositif de dissolution). La dissolution du principe actif dans le milieu peut être suivie par spectrophotométrie, de préférence à la longueur d'onde d'absorbance maximale. Un pH gastrique est typiquement un pH de 1 à 3.
« Au moins 60% du principe actif libéré en 45 minutes » englobe au moins 65%, au moins au moins 70%, au moins 75%, du principe actif est libéré en 45 minutes.
De préférence, au moins 65% du principe actif est libéré en 45 minutes.
La dispersion solide peut être, en outre, caractérisée en ce qu'au moins 55% du principe actif qu'elle contient est libéré en 30 minutes lorsqu'il est soumis à un test de dissolution *in vitro* comme décrit précédemment.

La dispersion solide peut être obtenue par l'une quelconque des méthodes décrites dans l'état de la technique. En particulier, la dispersion solide peut être obtenue par une première méthode dite « méthode par évaporation de solvant » comprenant la préparation d'une solution contenant l'excipient polymérique, le principe actif, et en option l'excipient pharmaceutique additionnel, dans un solvant, puis l'élimination dudit solvant de manière à obtenir la dispersion solide.

Le solvant est choisi de manière à solubiliser à la fois l'excipient polymérique, le principe actif et l'éventuel excipient additionnel. L'élimination du solvant peut être effectuée par toute méthode connue, par exemple, par évaporation par chauffage, par évaporation sous pression réduite ou sous vide, par lyophilisation ou par atomisation (spray-drying en anglais). L'atomisation consiste à disperser la solution sous la forme de fines gouttelettes dans un air chaud au moyen de buses. La taille finale des particules de la dispersion solide dépend de la taille des gouttelettes formées. Dans certains cas, la solution est vaporisée sur des particules d'un véhicule acceptable sur le plan pharmaceutique, typiquement un diluant, lesdites particules de véhicule étant mis en suspension dans un air chaud au sein d'un lit fluidisé. La dispersion solide finale se présente alors sous la forme d'un dépôt à la surface des particules de diluant.
La lyophilisation comprend, quant à elle, la congélation de la solution puis l'élimination du solvant par sublimation.
A titre alternatif, la dispersion solide peut être obtenue par une seconde méthode dite « méthode par fusion » dans laquelle un mélange, comprenant l'excipient polymérique et le principe actif, à l'état fondu est préparé puis solidifié de manière à obtenir la dispersion solide. Le mélange peut comprendre, en outre, un excipient pharmaceutique additionnel. Le mélange peut être formé selon plusieurs méthodes. Par exemple, on prépare un mélange contenant l'excipient polymérique, le principe actif (et éventuellement l'excipient additionnel) puis on chauffe ce mélange sous agitation de manière à promouvoir la fusion de l'excipient polymérique. A titre alternatif, on peut chauffer, dans un premier temps, l'excipient polymérique de manière à le fondre puis on ajoute, sous agitation, le principe actif et éventuellement l'excipient pharmaceutique additionnel. La température de chauffage est généralement supérieure à la température de transition vitreuse (Tg) de l'excipient polymérique. De manière préférée, le principe actif et l'excipient additionnel optionnel sont solubles dans l'excipient polymérique fondu.
Le mélange peut être préparé dans un réacteur traditionnel muni d'un moyen de chauffage et éventuellement d'un moyen de refroidissement. A titre alternatif, on peut utiliser une extrudeuse munie d'un moyen de chauffage. L'utilisation d'une extrudeuse couplée à un moyen de découpage ou de moulage de l'extrudat permet l'obtention de formes de dosage, par exemple de comprimés, directement en sortie de procédé, sans étape ultérieure de mise en forme.

La solidification de la dispersion solide peut être réalisée en laissant revenir le mélange comprenant l'excipient polymérique fondu et le principe actif à température ambiante, ou en le refroidissant. La solidification peut être également obtenue par atomisation (spray-drying) si la viscosité du mélange le permet.
Enfin, la dispersion solide peut être préparée par une méthode basée sur l'utilisation d'un fluide supercritique, par exemple le CO₂ supercritique. La dispersion solide peut être préparée par un procédé connu sous le nom d' « expansion rapide d'une solution supercritique » (en anglais Rapid Expansion of Supercritical Solution - RESS). Dans ce procédé, le fluide supercritique est utilisé en tant que solvant pour solubiliser le principe actif et l'excipient polymérique (et l'éventuel excipient additionnel). Cette solution supercritique est ensuite atomisée dans une chambre d'expansion sous faible pression. La faible pression présente dans la chambre d'expansion conduit à la formation de particules de dispersion solide. Cette technique est conceptuellement proche de la méthode dite par « solvant », le fluide supercritique remplaçant le solvant liquide.
A titre alternatif, le fluide supercritique peut être utilisé en tant qu'anti-solvant. Dans ce cas une solution contenant l'excipient polymérique et le principe actif (et l'éventuel excipient additionnel) est pulvérisée dans une enceinte contenant un fluide supercritique. La diffusion du fluide supercritique dans les gouttelettes provoque la précipitation de la dispersion solide. Un tel procédé est généralement connu sous le nom de « Précipitation par un anti-solvant compressé » (en anglais Précipitation with Compressed Anti-solvent - PCA).
Dans un mode de réalisation préféré, la dispersion solide est préparée par la méthode par évaporation de solvant ou par la méthode par fusion telle que précédemment décrites. Ainsi, la présente invention a également pour objet une méthode de préparation d'une dispersion solide comprenant les étapes consistant à :
a) préparer une solution comprenant l'excipient polymérique et le principe actif dans un solvant, puis éliminer ledit solvant de manière à obtenir la dispersion solide ; ou
b) préparer un mélange comprenant l'excipient polymérique à l'état fondu et le principe actif puis solidifier ledit mélange de manière à obtenir la dispersion solide.
L'aternative a) correspond à la méthode de préparation par solvant alors que l'alternative b) correspond à la méthode de préparation par fusion.

La méthode par évaporation de solvant (alternative a) est généralement utilisée lorsqu'il existe un solvant capable de solubiliser à la fois le principe actif et l'excipient polymérique, et si présent, l'excipient additionnel.
Dans ce cas, l'excipient polymérique peut être choisi parmi le groupe constitué par les polyvinylpyrrolidones, les copolymères de N-vinylpyrrolidone (par exemple les copovidones) et leurs combinaisons. Le solvant peut être l'acétone, un alcool, par exemple le méthanol, l'éthanol, l'isopropanol, 2-méthyl-2-propanol, isobutanol, et le tert-butanol, le dichlorométhane, le chloroforme, ou encore un ester tel que l'acétate d'éthyle ou un mélange de ceux-ci. Le rapport massique « excipient polymérique/principe actif » dans une dispersion solide obtenue par la méthode par évaporation de solvant est généralement de 1 à 20.
La méthode par fusion (alternative b) est, quant à elle, de préférence utilisée lorsque l'excipient polymérique présente une température de transition vitreuse basse - typiquement inférieure à 100°C. A titre d'exemple, on peut préparer la dispersion solide par la méthode dite de fusion lorsque l'excipient polymérique est un polyéthylène glycol.
Le rapport massique « excipient polymérique/principe actif » dans une dispersion solide obtenue par la méthode par fusion peut être de 20 à 50, de manière préférée de 20 à 30.
La méthode de préparation de la dispersion solide selon l'invention peut comprendre des étapes supplémentaires, telles que des étapes de broyage, de tamisage ou encore de moulage de la dispersion solide.

### Composition pharmaceutique selon l'invention

La dispersion solide selon l'invention est destinée principalement à une utilisation thérapeutique ou contraceptive. A cette fin, elle peut être administrée directement ou insérée dans un dispositif d'administration tel qu'un anneau vaginal, un patch, un stérilet ou un implant.
La dispersion solide selon l'invention peut en outre être intégrée dans une composition pharmaceutique. Ainsi, un objet supplémentaire de la présente invention est une composition pharmaceutique comprenant une dispersion solide telle que définie précédemment et au moins un excipient acceptable sur le plan pharmaceutique. L'homme du métier saura choisir le ou les excipients à associer à la dispersion solide en fonction de la forme finale de la composition pharmaceutique, de la voie d'administration souhaitée et du profil de libération du principe actif recherché. A cette fin, l'homme du métier pourra se référer aux ouvrages de référence suivants : Remington: The Science and Practice of Pharmacy (Lippincott Williams & Wilkins; Twenty first Edition, 2005) et, Handbook of Pharmaceuticals Excipients, American Pharmaceutical Association (Pharmaceutical Press; 6th revised édition, 2009). La composition pharmaceutique et la dispersion solide selon l'invention peuvent être administrées par toute voie, en particulier par voie orale, buccale, nasale, sublinguale, vaginale, intrautérine, rectale, transdermale ou par voie parentérale, par exemple par injection intraveineuse. Les voies d'administration préférées sont les voies buccale, orale, intrautérine et vaginale. La composition pharmaceutique selon l'invention peut se présenter sous toute forme, par exemple sous la forme d'un comprimé, d'une poudre, d'une gélule, d'une pilule, d'un suppositoire, d'une ovule, d'une suspension, d'un sirop, d'un gel, d'une pommade, d'une émulsion, d'un lyophilisat ou d'un film orodispersible. La voie d'administration et la forme galénique de la composition pharmaceutique pourront dépendre de l'effet thérapeutique ou contraceptif recherché.
Dans certains modes de réalisation, la composition pharmaceutique selon l'invention peut être intégrée dans un dispositif permettant l'administration prolongée du principe actif. La composition pharmaceutique pourra être, en particulier, intégrée dans un anneau vaginal, dans un patch, par exemple un patch transdermique ou muco-adhésif, dans un stérilet, ou dans un implant, par exemple un implant de type contraceptif. Pour des exemples d'anneaux vaginaux adaptés à la mise en oeuvre de l'invention, on pourra se référer à la demande WO2006/10097.
Dans des modes de réalisation supplémentaires, la composition pharmaceutique selon l'invention se présente sous forme solide. De préférence, la composition pharmaceutique selon l'invention est solide et destinée à une administration orale.
Dans certains modes de réalisation, la composition pharmaceutique selon l'invention est caractérisée en ce que l'excipient acceptable sur le plan pharmaceutique est choisi parmi le groupe constitué par un diluant, un liant, un agent d'écoulement, un lubrifiant, un agent désintégrant et leurs mélanges.
Un diluant au sens de la présente invention peut être un ou plusieurs composés capables de densifier le principe actif (ici la dispersion solide) pour obtenir la masse désirée. Les diluants englobent les phosphates minéraux, les monosaccharides et les polyols tels que le xylitol, le sorbitol, le lactose, le galactose, le xylose ou le mannitol, les disaccharides tels que le saccharose, les oligosaccharides, les polysaccarides tels que la cellulose et ses dérivés, les amidons, et les mélanges. Le diluant peut être sous forme anhydre ou hydraté.

A titre d'exemple, le diluant peut être choisi parmi la cellulose microcristalline, le mannitol, le lactose et leurs mélanges.
Le liant peut être un ou plusieurs composés capables d'améliorer l'agrégation du principe actif avec le diluant. On peut citer, à titre d'exemple, l'hydroxypropyl cellulose, l'hydroxypropylméthyl cellulose, la povidone (polyvinylpyrrolidone), les copolymères de N-vinyl-2-pyrrolidone et d'acétate de vinyle (copovidone) et leurs mélanges.
Le lubrifiant peut être un ou plusieurs composés capables d'empêcher les problèmes liés à la préparation des formes galéniques sèches, comme les problèmes de collage et/ou de grippage qui surviennent au niveau des machines lors de la compression ou du remplissage. Les lubrifiants préférés sont des acides gras ou des dérivés d'acides gras comme le stéarate de calcium, le monostéarate de glycéryle, le palmitostéarate de glycéryle, le stéarate de magnésium, le stéarate de zinc, ou l'acide stéarique, les polyalkylèneglycols, notamment le polyéthylèneglycol, le benzoate de sodium ou le talc.
Les lubrifiants préférés selon l'invention sont les sels de stéarates et leurs mélanges. Un lubrifiant adapté est par exemple le stéarate de magnésium.
L'agent d'écoulement éventuellement employé peut être choisi parmi les composés qui contiennent du silicium, par exemple, le talc, la silice colloïdale anhydre ou de la silice précipitée.
Le désintégrant peut être utilisé pour améliorer la libération du principe actif. Il peut être choisi parmi la polyvinyl pyrrolidone réticulée (crospovidone), la carboxyméthyl cellulose réticulée (telle que la croscarmellose de sodium) ou non-réticulée, les amidons et leurs mélanges. Le désintégrant est de préférence choisi dans le groupe constitué par une croscarmellose sodique, une crospovidone et leurs mélanges.

Dans certains modes de réalisation, la composition selon l'invention comprend :
- 0,5% à 95% de la dispersion solide telle que définie précédemment,
- 5% à 95 % de diluant, et
- 0% à 5% de lubrifiant,
les pourcentages étant exprimés en poids par rapport au poids total de la composition.
La composition selon l'invention peut être, en outre, caractérisée en ce qu'elle comprend de 0% à 20% en poids d'un agent liant, 0% à 10% d'agent désintégrant, et de 0% à 5% en poids d'un agent d'écoulement.
De préférence, la composition selon l'invention comprend :
- 10% à 95% en poids de dispersion solide telle que définie précédemment,
- 5% à 90% en poids de diluant, et
- 0% à 5% en poids de lubrifiant.
A titre d'exemple, la composition pharmaceutique selon l'invention peut comprendre de :
- 4% à 25% en poids de dispersion solide
- 70% à 90% en poids de diluant,
- 0,5% à 8% en poids d'agent désintégrant, et
- 0,1% à 5% d'un lubrifiant,
les pourcentages étant exprimés en poids par rapport au poids total de la composition.
Un exemple supplémentaire est une composition pharmaceutique comprenant de :
- 35% à 60% en poids de dispersion solide,
- 35% à 60% en poids de diluant,
- 0,5% à 8% en poids d'agent désintégrant, et
- 0,1% à 5% d'un lubrifiant,
les pourcentages étant exprimés en poids par rapport au poids total de la composition.

La composition pharmaceutique selon l'invention peut comprendre, en outre, un ou plusieurs excipients additionnels aux excipients précédemment cités. Le ou les excipients additionnels peuvent être choisis parmi le groupe constitué par les surfactants tels que le SDS, les agents d'enrobage tels que les agents d'enrobage à base d'alcool polyvynilique ou d'hydroxypropylmethylcellulose, les pigments tels que l'oxyde d'aluminium ou l'oxyde de fer, les arômes, les agents mouillants, les cires, les agents dispersants, les stabilisants et les conservateurs.
La composition pharmaceutique selon l'invention pourra être préparée selon l'une quelconque des méthodes couramment utilisées en galénique. Ces méthodes comprennent typiquement le mélange de la dispersion solide selon l'invention avec un ou plusieurs excipients puis la mise en forme du mélange obtenu. A titre d'exemple, lorsqu'elle se présente sous la forme d'un comprimé, la composition pharmaceutique selon l'invention peut être préparée, par compression directe ou par compression après granulation par voie sèche ou humide.
A titre alternatif, la composition pharmaceutique peut être préparée selon un procédé comprenant une étape de formation d'un dépôt de dispersion solide selon l'invention sur les particules de diluant, typiquement par vaporisation.

La dispersion solide présente dans la composition pharmaceutique selon l'invention peut avoir l'une quelconque des caractéristiques décrites dans la présente description. En particulier, la dispersion solide présente dans la composition pharmaceutique a une ou plusieurs (1, 2, 3, 4, 5, ou 6) des caractéristiques suivantes :
i. le principe actif est l'ulipristal acétate ou ses métabolites
ii. l'excipient polymérique est choisi parmi le groupe constitué par les polyvinylpyrrolidones, les copolymères de N-vinylpyrrolidone, tels que les copovidones, et leurs combinaisons,
iii. le rapport massique « excipient polymérique/principe actif » est compris dans une gamme allant de 1 à 50, de préférence de 1 à 30, voire de 1 à 20 et même de 1,5 à 10.
iv. la dispersion solide comprend, en outre, un surfactant, de préférence le SDS de préférence à une teneur inférieure à 10% en poids par rapport au poids total de la dispersion solide.
v. le principe actif est présent dans la dispersion solide essentiellement sous une forme amorphe
vi. au moins 60% du principe actif contenu dans la dispersion solide est libéré en 45 minutes lorsque ladite dispersion solide est soumise à un test de dissolution *in vitro,* de préférence dans les conditions suivantes :
   - dispositif: dispositif de dissolution à pales,
   - échantillon : gélule en gélatine dure contenant une quantité de dispersion solide correspondant à 30 mg de principe actif,
   - milieu de dissolution : 900 ml d'une solution aqueuse tamponnée à pH gastrique comprenant 0,1% de SDS,
   - température : 37±0,5 °C, et
   - vitesse de rotation des pales : 50 tours par minute (rpm)

Comme mentionné précédemment, la composition selon l'invention peut se présenter sous la forme d'une poudre, d'une granule, d'un comprimé pelliculé ou non pelliculé, ou d'une gélule, et est de préférence destinée à une administration orale. Dans certains modes de réalisation, la composition pharmaceutique selon l'invention se présente sous la forme d'un comprimé non-pelliculé destiné à une administration orale.

La composition selon l'invention peut être une composition pharmaceutique à libération contrôlée, immédiate, prolongée ou retardée. De préférence, la composition selon l'invention est une composition à libération immédiate.
On entend par une « composition à libération immédiate », une composition pharmaceutique caractérisée en ce qu'au moins 60%, de préférence au moins 75%, du principe actif initialement contenu dans une unité de dose de la composition pharmaceutique est libéré en 45 minutes lorsque ladite unité de dose est soumise à un test de dissolution in vitro, par exemple selon Pharmacopée Européenne §2.9.3, de préférence, dans les conditions suivantes :
- dispositif de dissolution à pales,
- milieu de dissolution : solution aqueuse tamponnée à pH gastrique contenant 0,1% de SDS
- température : 37±0.5°C, et
- vitesse de rotation : 50 rpm.
Le volume du milieu de dissolution dépend de la quantité de principe actif contenue dans l'unité de dose. Pour une unité de dose comprenant 5 mg de principe actif, on utilise 500 ml de milieu de dissolution. Pour une unité de dose comprenant 30 mg de principe actif, on utilise 900 ml de milieu de dissolution.

De manière générale, la composition pharmaceutique comprend de 1 mg à 100 mg de principe actif par unité de dose, de préférence de 1 mg à 40 mg, voire de 2 mg à 30 mg, de principe actif par unité de dose. La dose de principe actif est fonction de l'effet thérapeutique ou contraceptif et du schéma d'administration recherchés. Par exemple pour certaines applications, la quantité de principe actif par unité de dose peut être comprise dans une gamme allant de 1 mg à 5 mg.
En contraception d'urgence, le principe actif, de préférence l'UPA, peut être présent à hauteur de 20 mg à 40 mg par unité de dose. En contraception régulière, le principe actif, de préférence l'UPA, peut être présent à hauteur de 1 mg à 5 mg par unité de dose.
Pour des utilisations thérapeutiques tels que le traitement des fibromes utérins, le principe actif, de préférence l'UPA, peut être présent à hauteur de 3 mg à 15 mg par unité de dose. La dose en principe actif et le schéma d'administration pourra également dépendre des paramètres personnels du patient, en particulier de son poids, son âge, son sexe, son état de santé général, son régime alimentaire, des pathologies dont il souffre...

Enfin, la composition pharmaceutique selon l'invention peut comprendre un principe actif supplémentaire. Cet actif supplémentaire peut exercer une action distincte de celle du modulateur sélectif du récepteur à la progestérone, de préférence l'ulipristal acétate, ou de ses métabolites. Il peut également renforcer l'effet thérapeutique du modulateur sélectif du récepteur à la progestérone, de préférence l'ulipristal acétate, ou de ses métabolites.

### Utilisations thérapeutiques ou contraceptives de la dispersion solide et de la composition pharmaceutique selon la présente invention

Dans un aspect supplémentaire, la présente invention a également pour objet une dispersion solide ou une composition pharmaceutique telles que décrites précédemment pour une utilisation en tant que médicament. La dispersion solide ou la composition selon l'invention sont particulièrement adaptés pour une utilisation en tant que contraceptif régulier ou contraceptif d'urgence. Ils peuvent être également utilisés pour le traitement ou la prévention de troubles hormonaux, gynécologiques ou endocriniens tels que la maladie de Cushing. La composition ou la dispersion solide selon l'invention peuvent être utilisées, en particulier, dans le traitement ou la prévention d'un trouble gynécologique, de préférence affectant l'utérus, y compris les troubles gynécologiques bénins. Les troubles gynécologiques englobent, sans y être limités, les fibromes utérins et ses symptômes, l'adénomyose, l'endométriose, les douleurs associées à la dislocation de l'endomètre, les saignements utérins excessifs.
Un objet supplémentaire de l'invention est l'utilisation d'une dispersion solide selon l'invention, de préférence contenant de l'ulipristal acétate en tant que principe actif, pour la préparation d'un contraceptif ou pour la préparation d'un médicament destiné au traitement ou à la prévention de l'une quelconque des pathologies ci-dessus citées.
L'invention a également pour objet une méthode de contraception comprenant l'administration à une patiente d'une dose contraceptive de la dispersion solide ou de la composition pharmaceutique, de préférence contenant de l'ulipristal acétate en tant que principe actif.
On entend par « méthode de contraception » une méthode permettant d'éviter la survenue d'une grossesse chez une patiente en âge de procréer. Dans le cas d'espèce, il peut s'agir d'une méthode de contraception d'urgence. Dans ce cas, une dose unique est de préférence administrée à la patiente dans un intervalle de temps adéquate après le rapport non ou mal protégé, en général dans les 120 h suivant le rapport non ou mal protégé. La méthode de contraception peut être également une méthode de contraception régulière, dans laquelle la composition ou la dispersion solide sont administrées de manière chronique et cyclique à la patiente ou de manière continue à l'aide d'un dispositif tel qu'un implant ou un anneau vaginal. A titre alternatif, la méthode de contraception peut être une méthode de contraception dite « à la demande » telle que décrite dans la demande internationale WO2010/119029. Enfin, l'invention a également pour objet une méthode de traitement d'une maladie ou d'un trouble chez un patient comprenant l'administration d'une dose efficace sur le plan thérapeutique de la dispersion solide ou de la composition pharmaceutique, de préférence contenant de l'ulipristal acétate en tant que principe actif, au patient, de préférence de sexe féminin. La méthode thérapeutique selon l'invention concerne, de préférence, l'un quelconque des maladies ou troubles cités ci-dessus.
Il va de soi que pour la mise en oeuvre des méthodes et utilisations ci-dessus décrites, la dispersion solide et la composition pharmaceutique selon l'invention peuvent comprendre une ou plusieurs des caractéristiques détaillées dans la présente description.

Les exemples ci-après ont pour but d'illustrer plus pleinement l'invention sans pour autant en limiter la portée.

### EXEMPLES

### Exemple 1 : préparation et évaluation de différentes dispersions solides

### 1. Préparation de dispersions solides selon l'invention

### a) Préparation d'une dispersion solide par la méthode par fusion

On prépare une dispersion solide comprenant de l'ulipristal acétate en tant que principe actif et un polyethylène glycol (PEG-4000) en tant qu'excipient polymérique selon un rapport massique « excipient polymérique/UPA » de 960/40 de la manière suivante :
- le polyéthylène glycol est chauffé jusqu'à ce qu'il fonde complètement.
- De l'UPA micronisé est rajouté au PEG fondu sous agitation. Le mélange est chauffé et maintenu sous agitation jusqu'à dissolution complète de l'UPA,
- Le mélange est ramené à température ambiante sous agitation,
- La dispersion solide obtenue est éventuellement broyée ou micronisée de manière à obtenir la granulométrie souhaitée.
D'autres dispersions solides par la méthode par fusion ont été également préparées :
- UPA/gelucire® 50/13 (Gattefossé) selon un rapport massique 40/960
- UPA/PEG-1450/SDS selon un rapport massique 40/910/50.

### b) Préparation d'une dispersion solide par la méthode dite par évaporation de solvant

Les dispersions solides suivantes ont été préparées par la méthode dite par solvant :
- Dispersion solide UPA/Copovidone selon rapport massique 3/7,
- Dispersion solide UPA/Copovidone/SLS selon rapport massique 3/7/1,
- Dispersion solide UPA/Povidone selon rapport massique 3/7,
- Dispersion solide UPA/Povidone/SDS selon un rapport massique 3/6/1
- Dispersion solide UPA/Povidone/ SDS selon un rapport massique 3/5/2

L'UPA est de l'ulipristal acétate micronisé. La povidone correspond au produit commercial Plasdone® K-29-32 commercialisée par la société ISP. La copovidone correspond, quant à elle, au produit Plasdone® S630 commercialisée par ISP.

Ces dispersions solides ont été préparées de la manière suivante :
- L'excipient polymérique a été solubilisé dans l'éthanol à température ambiante,
- L'UPA, (et éventuellement le SDS si présent), a été ajouté sous agitation à cette solution. La solution a été maintenue sous agitation et à température ambiante jusqu'à dissolution complète de l'UPA.
- Le solvant (éthanol) a été ensuite lentement évaporé sous pression réduite à l'aide d'un évaporateur rotatif jusqu'à l'obtention de la dispersion solide.

### 2. Profils de dissolution in vitro des dispersions solides préparées

### a) Matériel et méthode

Pour chaque dispersion solide obtenue par la méthode dite par évaporation de solvant (§ 1.b) ci-avant), on a préparé des gélules en gélatine dure contenant une quantité de de dispersion solide correspondant à 30 mg d'UPA par gélule.

Les profils de dissolution ont été obtenus comme suit :

Chaque gélule contenant une dispersion solide a été placée dans un bol du dispositif de dissolution à pales contenant 900 ml d'un milieu de dissolution. Le milieu de dissolution est une solution aqueuse tamponnée à pH gastrique et comprenant 0,1% en poids de SDS.

Les conditions de mise en oeuvre des dissolutions *in vitro* sont les suivantes :
- Vitesse de rotation des pales : 50 tours par minute (rpm)
- Température : 37°C ± 0,5°C

La dissolution de l'UPA a été suivie par spectrophotométrie.

A titre d'expérience témoin, on a utilisé une gélule en gélatine contenant 30 mg d'ulipristal acétate micronisé. Pour chaque dispersion solide évaluée, l'expérience de dissolution a été reproduite 3 fois.

### b) Résultats

### Dispersions solides préparées par fusion

Le tableau 1 ci-dessous et la Figure 5 montrent les résultats de dissolution obtenus pour ces dispersions solides. Les pourcentages de dissolution sont exprimés par rapport à la quantité initiale d'UPA contenue dans chaque gélule.

**Tableau 1 : Résultats des essais de dissolution in vitro pour les dispersions solides préparées par fusion. Expérience témoin : UPA micronisé.**

| Pourcentages d'UPA libéré exprimés par rapport à la quantité initiale d'UPA contenue par gélule. (Valeurs moyennes sur 3 expériences) | | | | |
|---|---|---|---|---|
| Temps (min) | UPA micronisé | UPA/PEG-4000 | UPA/Gélucire | UPA/PEG-1450/SDS |
| 0 | 0 | 0,00 | 0,00 | 0,00 |
| 1 | 0,23 | 0,70 | 0,00 | 0,20 |
| 5 | 5,69 | 3,80 | 0,30 | 5,80 |
| 7,5 | 15,28 | 8,10 | 0,70 | 13,90 |
| 10 | 21,73 | 12,40 | 1,10 | 28,50 |
| 15 | 29,46 | 28,10 | 1,80 | 62,20 |
| 20 | 35,2 | 49,90 | 2,60 | 85,40 |
| 30 | 42,14 | 89,50 | 4,20 | 93,50 |
| 45 | 49,98 | 104,50 | 6,50 | 94,70 |
| 60 | 55,62 | 105,30 | 8,70 | 95,10 |

De manière remarquable, les dispersions solide d'UPA et d'excipient polymérique présentent une vitesse de libération de l'UPA bien plus élevée que celle observée pour les gélules comprenant de l'UPA micronisée. En revanche, l'utilisation d'un autre type d'excipient de dispersion solide (gélucire) a un impact très négatif sur la vitesse de dissolution. Ceci illustre l'effet spécifique découlant de l'utilisation d'un excipient polymérique.

### Dispersions solides préparées par la méthode par évaporation de solvant

Le tableau 2 ci-dessous et la Figure 1 montrent les résultats de dissolution obtenus pour les dispersions solides ne comprenant pas de SDS. Les pourcentages de dissolution sont exprimés par rapport à la quantité initiale d'UPA contenue dans chaque gélule.

**Tableau 2 : Résultats des essais de dissolution in vitro pour les dispersions solides préparées. Expérience témoin : UPA micronisé.**

| | Pourcentages d'UPA libéré exprimés par rapport à la quantité initiale d'UPA contenue par gélule. (Valeurs moyennes sur 3 expériences) | | |
|---|---|---|---|
| Temps (min) | Ulipristal micronisé | UPA/Povidone 3/7 | UPA/copovidone 3/7 |
| 0 | 0 | 0,00 | 0,00 |
| 1 | 0,23 | 0,10 | 0,00 |
| 5 | 5,69 | 7,80 | 8,00 |
| 7,5 | 15,28 | 18,70 | 19,50 |
| 10 | 21,73 | 28,10 | 28,40 |
| 15 | 29,46 | 44,10 | 41,80 |
| 20 | 35,2 | 54,80 | 50,10 |
| 30 | 42,14 | 66,50 | 60,80 |
| 45 | 49,98 | 74,40 | 71,30 |
| 60 | 55,62 | 77,50 | 76,50 |

Ces résultats montrent que les dispersions solides d'UPA et de povidone ou de copovidone ont une vitesse de libération de l'UPA beaucoup plus élevée que celle observée pour l'UPA micronisé. De manière remarquable, le pourcentage d'ulipristal acétate libéré dans le milieu à t = 20 min est d'environ 66% pour une gélule comprenant la dispersion solide UPA/povidone et d'environ 61% pour une gélule comprenant la dispersion solide UPA/copovidone alors qu'elle n'est que d'environ 42% pour une gélule contenant de l'ulipristal acétate micronisé.
L'effet d'un excipient additionnel - à savoir le SDS- sur la vitesse de dissolution *in vitro* de l'UPA a été évalué. Le tableau 3 ci-dessous et la Figure 2 montrent les résultats de dissolution obtenus pour les dispersions solides comprenant de SDS. Les pourcentages de dissolution sont exprimés par rapport à la quantité initiale d'UPA contenue dans chaque gélule.

**Tableau 3 : Résultats des essais de dissolution in vitro pour les dispersions solides préparées. Expérience témoin : UPA micronisé.**

| | Pourcentages d'UPA libéré exprimés par rapport à la quantité initiale d'UPA contenue par gélule. (Valeurs moyennes sur 3 expériences) | | |
|---|---|---|---|
| Temps (min) | UPA micronisé | UPA/povidone/SDS 3/6/1 | UPA/povidone/SDS 3/5/2 |
| 0 | 0 | 0,00 | 0 |
| 1 | 0,23 | 1,80 | - |
| 5 | 5,69 | 11,10 | 18,80 |
| 7,5 | 15,28 | 16,90 | 28,90 |
| 10 | 21,73 | 21,10 | 38,40 |
| 15 | 29,46 | 27,80 | 75,00 |
| 20 | 35,2 | 33,50 | 92,00 |
| 30 | 42,14 | 48,50 | 102,30 |
| 45 | 49,98 | 64,40 | 107,90 |
| 60 | 55,62 | 70,80 | 111,00 |

Les dispersions solides UPA/povidone/SDS présentent une vitesse de dissolution in vitro de l'UPA améliorée par rapport à l'UPA micronisée, en particulier lorsque le SDS est présent à hauteur de 20% en poids.
Toutefois, de manière quelque peu surprenante, la dispersion solide UPA/povidone/SDS 3/6/1 est moins efficace que la dispersion solide UPA/povidone 3/7.

### 3. Profil de cristallinité de l'UPA présent dans les dispersions solides

Les dispersions solides obtenues ont été analysées par diffractométrie sur poudre par rayons X à l'aide du dispositif Phillips PW-1050 X Ray diffractomètre.

Le spectre de diffraction de la dispersion solide UPA/Povidone/SDS 3/6/1 (voir Figure 3A) présente des pics de diffraction de faible intensité suggérant que l'UPA est présent sous une forme majoritairement amorphe dans la dispersion solide. De manière notable, cette dispersion solide est stable puisqu'aucune conversion significative de l'UPA amorphe vers une forme cristalline n'est observée au bout d'1 mois de stockage à température ambiante (voir Figure 3B).

En revanche, une augmentation de la teneur de SDS dans la dispersion solide provoque l'apparition d'UPA sous forme cristalline comme l'atteste le spectre de diffraction de la dispersion solide UPA/Povidone/SDS 3/5/2 (voir Figure 3B).

### Exemple 2 : Compositions pharmaceutiques intégrant une dispersion solide selon l'invention

Les tableaux 3 et 4 ci-après présentent des exemples de composition pharmaceutique selon l'invention. Ces compositions pharmaceutiques peuvent être obtenues en mélangeant une dispersion solide selon l'invention avec différents excipients, puis en mettant en forme ledit mélange par compression directe de manière à obtenir des comprimés.

**Tableau 3 : Exemple d'une composition selon l'invention à 5 mg d'UPA**

| Ingrédients | Fonction | % en poids | mg/comprimé |
|---|---|---|---|
| Dispersion solide UPA/povidone 3/7 | Matrice de principe actif | 11,1 | 16,7 (soit 5 mg d'UPA) |
| Cellulose microcristalline | Diluant | 54,4 | 81,6 |
| Mannitol | Diluant | 29,0 | 43,5 |
| Crospovidone | Désintégrant | 5,0 | 7,5 |
| Stéarate de magnésium | Lubrifiant | 0,5 | 0,75 |
| Total | | | 150 |

Cette composition peut être utilisée, par exemple, pour le traitement des fibromes utérins.

**Tableau 4 : Exemple d'une composition selon l'invention à 30 mg d'UPA**

| Ingrédients | Fonction | % en poids | mg/comprimé |
|---|---|---|---|
| Dispersion solide UPA/povidone 3/7 | Matrice de principe actif | 48,0 | 100 |
| Mannitol | Diluant | 48,0 | 100 |
| Crospovidone | Désintégrant | 3,6 | 7,5 |
| Stéarate de magnésium | Lubrifiant | 0,4 | 0,75 |
| Total | | | 208,25 |

Cette composition peut être utilisée, par exemple, en contraception d'urgence.

## Revendications

1. Dispersion solide comprenant :
- un principe actif choisi dans le groupe constitué par la 17α-acétoxy-11β-[4-N-méthylamino-phényl]-19-norprégna-4,9-diène-3,20-dione, la 17α-acétoxy-11β-[4-amino-phényl]-19-norprégna-4,9-diène-3,20-dione, l'ulipristal acétate et leurs mélanges,
- un excipient polymérique acceptable sur le plan pharmaceutique choisi dans le groupe constitué par les polyvinylpyrrolidones, les copolymères de N-vinylpyrrolidone, et leurs combinaisons, et
- un surfactant choisi parmi les sels de dodécyl sulfate.

2. Dispersion solide selon la revendication 1 **caractérisée en ce que** le principe actif est l'ulipristal acétate

3. Dispersion solide selon l'une des revendications 1 et 2 **caractérisée en ce que** l'excipient polymérique acceptable sur le plan pharmaceutique est choisi dans le groupe constitué par une polyvinylpyrrolidone, une copovidone et leurs combinaisons.

4. Dispersion solide selon l'une quelconque des revendications 1 à 3 **caractérisée en ce que** le rapport massique « excipient polymérique/principe actif » est compris dans une gamme allant de 1 à 50, de préférence de 1 à 30.

5. Dispersion solide selon l'une quelconque des revendications 1 à 4 **caractérisée en ce que** le rapport massique « principe actif/surfactant » est compris dans une gamme allant de 0,5 à 10.

6. Dispersion solide selon l'une quelconque des revendications 1 à 5 **caractérisée en ce que** le surfactant est choisi parmi les sels alcalins et alcalino-terreux du dodécylsulfate.

7. Dispersion solide selon la revendication 6, dans laquelle le surfactant est le dodécylsulfate de sodium.

8. Dispersion solide selon l'une quelconque des revendications 1 à 7, **caractérisée en ce qu'**elle se présente sous la forme d'un dépôt à la surface d'un véhicule acceptable sur le plan pharmaceutique.

9. Méthode de préparation d'une dispersion solide telle que définie dans l'une quelconque des revendications 1 à 8 **caractérisée en ce qu'**elle comprend les étapes consistant à :
a) préparer une solution comprenant l'excipient polymérique, le surfactant et le principe actif dans un solvant, puis éliminer ledit solvant de manière à obtenir la dispersion solide ; ou
b) préparer un mélange comprenant l'excipient polymérique à l'état fondu, le surfactant, et le principe actif puis solidifier ledit mélange de manière à obtenir la dispersion solide.

10. Composition pharmaceutique comprenant une dispersion solide telle que définie dans l'une des revendications 1 à 8 et un excipient acceptable sur le plan pharmaceutique, de préférence, choisi parmi le groupe constitué par un diluant, un liant, un agent d'écoulement, un lubrifiant, un agent désintégrant et leurs mélanges.

11. Composition pharmaceutique selon la revendication 10 **caractérisée en ce qu'**elle comprend de 1 mg à 100 mg, de préférence de 1 mg à 40 mg de principe actif par unité de dose.

12. Composition pharmaceutique selon la revendication 10 ou 11 **caractérisée en ce qu'**elle est adaptée à une administration par voie orale et se présente, de préférence, sous la forme d'une poudre, d'une granule, d'un comprimé pelliculé ou non pelliculé, ou d'une gélule.

13. Dispersion solide selon l'une quelconque des revendications 1 à 8 ou composition pharmaceutique selon l'une quelconque des revendications 10 à 12 pour une utilisation en tant que contraceptif.

14. Dispersion solide selon l'une quelconque des revendications 1 à 8 ou composition pharmaceutique selon l'une quelconque des revendications 10 à 12 pour une utilisation dans le traitement ou la prévention d'un trouble gynécologique, de préférence affectant l'utérus.

## Patentansprüche

1. Feststoffdispersion, umfassend:
- einen Wirkstoff, ausgewählt aus der Gruppe, bestehend aus 17α-Acetoxy-11β-[4-N-methylamino-phenyl]-19-norpregna-4,9-dien-3,20-dion, 17α-Acetoxy-11β-[4-N-aminophenyl]-19-norpregna-4,9-dien-3,20-dion, Ulipristalacetat und Gemischen davon,
- einen pharmazeutisch verträglichen polymeren Arzneimittelträger, ausgewählt aus der Gruppe, bestehend aus Polyvinylpyrrolidonen, Copolymeren von N-Vinylpyrrolidon und Kombinationen davon, und
- einen grenzflächenaktiven Stoff, ausgewählt aus Dodecylsulfat-Salzen.

2. Feststoffdispersion gemäß Anspruch 1, **dadurch gekennzeichnet, dass** der Wirkstoff Ulipristalacetat ist.

3. Feststoffdispersion gemäß einem der Ansprüche 1 und 2, **dadurch gekennzeichnet, dass** der pharmazeutisch verträgliche polymere Arzneimittelträger aus der Gruppe ausgewählt ist, bestehend aus einem Polyvinylpyrrolidon, einem Copovidon und Kombinationen davon.

4. Feststoffdispersion gemäß einem der Ansprüche 1 bis 3, **dadurch gekennzeichnet, dass** das Massenverhältnis "polymerer Arzneimittelträger/Wirkstoff" in einem Bereich von 1 bis 50, vorzugsweise von 1 bis 30, liegt.

5. Feststoffdispersion gemäß einem der Ansprüche 1 bis 4, **dadurch gekennzeichnet, dass** das Massenverhältnis "Wirkstoff/grenzflächenaktiver Stoff" in einem Bereich von 0,5 bis 10 liegt.

6. Feststoffdispersion gemäß einem der Ansprüche 1 bis 5, **dadurch gekennzeichnet, dass** der grenzflächenaktive Stoff aus Alkali- und Erdalkalisalzen von Dodecylsulfat ausgewählt ist.

7. Feststoffdispersion gemäß Anspruch 6, wobei der grenzflächenaktive Stoff Natriumdodecylsulfat ist.

8. Feststoffdispersion gemäß einem der Ansprüche 1 bis 7, **dadurch gekennzeichnet, dass** sie in Form einer Anlagerung auf der Oberfläche eines pharmazeutisch verträglichen Vehikels vorliegt.

9. Verfahren zur Herstellung einer wie in einem der Ansprüche 1 bis 8 definierten Feststoffdispersion, **dadurch gekennzeichnet, dass** es die Schritte umfasst, bestehend aus:
a) Herstellen einer Lösung, umfassend den polymeren Arzneimittelträger, den grenzflächenaktiven Stoff und den Wirkstoff in einem Lösemittel, anschließend Entfernen besagten Lösemittels, um die Feststoffdispersion zu erhalten; oder
b) Herstellen eines Gemisches, umfassend den polymeren Arzneimittelträger in geschmolzenem Zustand, den grenzflächenaktiven Stoff und den Wirkstoff, anschließend Verfestigen besagten Gemisches, um eine Feststoffdispersion zu erhalten.

10. Pharmazeutische Zusammensetzung, umfassend eine wie in einem der Ansprüche 1 bis 8 definierte Feststoffdispersion und einen pharmazeutisch verträglichen Arzneimittelträger, vorzugsweise ausgewählt aus der Gruppe, bestehend aus einem Verdünnungsmittel, einem Bindemittel, einem Fließmittel, einem Gleitmittel, einem Sprengmittel und Gemischen davon.

11. Pharmazeutische Zusammensetzung gemäß Anspruch 10, **dadurch gekennzeichnet, dass** sie 1 mg bis 100 mg, vorzugsweise 1 mg bis 40 mg, Wirkstoff pro Dosiseinheit umfasst.

12. Pharmazeutische Zusammensetzung gemäß Anspruch 10 oder 11, **dadurch gekennzeichnet, dass** sie für eine orale Verabreichung geeignet ist und vorzugsweise in Form eines Pulvers, eines Granulats, einer Filmtablette oder einer filmlosen Tablette oder einer Kapsel vorliegt.

13. Feststoffdispersion gemäß einem der Ansprüche 1 bis 8 oder pharmazeutische Zusammensetzung gemäß einem der Ansprüche 10 bis 12 für eine Verwendung als Kontrazeptivum.

14. Feststoffdispersion gemäß einem der Ansprüche 1 bis 8 oder pharmazeutische Zusammensetzung gemäß einem der Ansprüche 10 bis 12 für eine Verwendung in der Behandlung oder Prävention einer bevorzugt den Uterus betreffenden gynäkologischen Störung.

## Claims

1. A solid dispersion comprising:
- an active ingredient selected from the group consisting of 17α-acetoxy-11β-[4-N-methylaminophenyl]-19-norpregna-4,9-diene-3,20-dione, 17α-acetoxy-11β-[4-aminophenyl]-19-norpregna-4,9-diene-3,20-dione, ulipristal acetate and mixtures thereof,
- a pharmaceutically acceptable polymeric excipient selected from the group consisting of polyvinylpyrrolidones, N-vinyl-2-pyrrolidone copolymers, and combinations thereof, and
- a surfactant selected from dodecyl sulphate salts.

2. The solid dispersion according to claim 1 wherein the active ingredient is ulipristal acetate.

3. The solid dispersion according to claims 1 or 2 wherein the pharmaceutically acceptable polymeric excipient is selected from the group consisting of a polyvinylpyrrolidone, a copovidone, and combinations thereof.

4. The solid dispersion according to any one of claims 1 to 3 wherein the "polymeric excipient/active ingredient" weight ratio is included in a range from 1 to 50, preferably from 1 to 30.

5. The solid dispersion according to any one of claims 1 to 4 wherein the "active ingredient/surfactant" weight ratio is included in a range from 0.5 to 10.

6. The solid dispersion according to any one of claims 1 to 5 wherein the surfactant is selected from alkali metal salts and alkaline-earth metal salts of dodecyl sulphate.

7. The solid dispersion according to claim 6, wherein the surfactant is sodium dodecyl sulphate.

8. The solid dispersion according to any one of claims 1 to 7, which is in the form of a deposit at the surface of a pharmaceutically acceptable carrier.

9. The method for preparing a solid dispersion as defined in any one of claims 1 to 8, which comprises the steps consisting in:
a) preparing a solution comprising the polymeric excipient, the surfactant and the active ingredient in a solvent, then eliminating said solvent so as to obtain the solid dispersion; or
b) preparing a mixture comprising the polymeric excipient in the molten state, the surfactant and the active ingredient, then solidifying said mixture so as to obtain the solid dispersion.

10. A pharmaceutical composition comprising a solid dispersion as defined in one of claims 1 to 8 and a pharmaceutically acceptable excipient, preferably selected from the group consisting of a diluent, a binder, a flow agent, a lubricant, a disintegrant and mixtures thereof.

11. The pharmaceutical composition according to claim 10 which comprises from 1 mg to 100 mg, preferably from 1 mg to 40 mg, of active ingredient per dose unit.

12. The pharmaceutical composition according to claim 10 or 11, which is suitable for oral administration and is preferably in the form of a powder, a granule, a coated or uncoated tablet, or a capsule.

13. A solid dispersion according to any one of claims 1 to 8 or pharmaceutical composition according to any one of Claims 10 to 12 for use as a contraceptive.

14. A solid dispersion according to any one of claims 1 to 8 or a pharmaceutical composition according to any one of Claims 10 to 12, for use in the treatment or the prevention of a gynaecological disorder, preferably affecting the uterus.
